# EUROPEAN PATENT SPECIFICATION

(11) **EP 2 510 926 B1**
(45) Date of publication and mention of the grant of the patent: **28.03.2018**
(21) Application number: 10835373.1
(22) Date of filing: 08.12.2010
(51) Int. Cl.: A61K 31/132, A61K 9/08, A61P 35/00, A61P 35/02, A61P 7/00, A61P 43/00

(54) **USE OF SATURATED AMINES COMPOUNDS IN MANUFACTURE OF MEDICAMENTS FOR MOBILIZING PERIPHERAL BLOOD HEMATOPOIETIC STEM CELL**
VERWENDUNG GESÄTTIGTER AMINVERBINDUNGEN BEI DER HERSTELLUNG VON MEDIKAMENTEN ZUR MOBILISIERUNG BLUTBILDENDER STAMMZELLEN IM PERIPHEREN BLUT
EMPLOI D'AMINES SATURÉES DANS LA FABRICATION DE MÉDICAMENTS DESTINÉS À LA MOBILISATION DE CELLULES SOUCHES HÉMATOPOÏÉTIQUES DU SANG PÉRIPHÉRIQUE

(30) Priority: 08.12.2009 CN 200910241726
(43) Date of publication of application: 17.10.2012
(73) Proprietor: Institue of Transfusion Medicine, Academy of Military Medical Sciences, People's Liberation Army of China, Beijing 100850 (CN)
(72) Inventor: PEI, Xuetao, Beijing 100850 (CN); SHI, Wei, Beijing 100850 (CN); LI, Yanhua, Beijing 100850 (CN); REN, Xiangliang, Beijing 100850 (CN); LV, Yang, Beijing 100850 (CN); SHI, Shuangshuang, Beijing 100850 (CN); CHEN, Lin, Beijing 100850 (CN); NAN, Xue, Beijing 100850 (CN); YUE, Wen, Beijing 100850 (CN)
(74) Representative: Aamand, Jesper L.
(86) International application number: PCT/CN2010/001986
(87) International publication number: WO 2011/069336

(56) References cited:
- CN-A- 101 716 167
- US-A1- 2006 281 712
- SILVIA DEL PIERO ET AL: "Affinity of Tripodal and Linear Tetraamines for Silver(I) in Dimethyl Sulfoxide", JOURNAL OF SOLUTION CHEMISTRY, KLUWER ACADEMIC PUBLISHERS-PLENUM PUBLISHERS, NE, vol. 37, no. 4, 19 March 2008 (2008-03-19) , pages 543-551, XP019577273, ISSN: 1572-8927
- YU, SHOUCHENG ET AL: 'Using various kinds of mobilized agent scheme in malignant lymphoma combined autologous peripheral stem cell transplantation and clinical effective observation' SHANGHAI MEDICAL SCIENCE(CHINESE) 19 October 1996, pages 590 - 594
- LIU, CHUANFANG ET AL: 'Effect of anti-CD49d monoclonal antibody on mobilization of CD34 positive cell into peripheral blood in mice' JOURNAL OF EXPERIMENTAL HEMATOLOGY(CHINESE) vol. 12, no. 1, 2004, pages 59 - 62

## Description

### Technical Field

The invention relates to a new use of a compound, particularly a saturated amine compound (Code No. T_{A01}) in the manufacture of peripheral blood hematopoietic stem cell mobilizing agents.

### Background

Bone marrow (BM) of adults is a place for regeneration of blood cells. It is also the storage place of stem cells or progenitor cells, such as endothelial progenitor cells forming blood vessels and mesenchymal stem cells that can differentiate into adipocytes, chondrocytes and osteoblasts. Early studies showed that hematopoietic progenitor cells were increased in the peripheral blood of the patients recovered from chemotherapy. Thus, it was recognized that hematopoietic stem and progenitor cells can be induced to move out of BM, and the process is called stem cell mobilization. The mobilized cells can aggregate as well as return to BM to produce blood cells. These hematopoietic cells induced into the blood can be used for transplantation. Latest researches on induction and isolation of stem cells showed that quantitative and qualitative research and potential function of hematopoietic stem cells (HSCs), and therapeutic efficiency of peripheral blood HSC transplantation are currently hotly studied.

### Effect of granulocyte colony stimulating factor (G-CSF) on peripheral blood stem cell (PBSC) induction

Various factors have the ability to mobilize stem cells into peripheral blood, suggesting that there are multiple mechanisms that regulate stem cell mobilization. A certain number of stem cells and (or) stem cells with certain properties can be induced to transplant under the synergistic action of multiple factors. G-CSF is the main inducer applied in clinical stem cell transplantation. It can induce PBSCs more easily as compared with BM stem cells and cord blood stem cells, make neutrophilic granulocytes and platelets recover faster and demand less for platelet transfusion, promote lymphocyte reconstitution, and reduce related inflammation incidence and fatality rate. In comparison with G-CSF, chemically induced PBSC transplantation needs more recovery time for blood cells, induces more complications such as fever, and requires more blood transfusion. Besides, chemically harvested PBSCs have already been inactive when used for allogenic transplantation. PBSC collection avoids the invasive operation and regular anesthesia related risks of BM collection. For transplant pairing and HLA matching, 1-3 components of the PBSCs can be eliminated, and the haploidentity can be assured. Stem cell transplantation based on G-CSF has good graft tolerance, although spleen rupture happens occasionally. Most donors can complete collection of stem cells that are sufficient for transplantation. Although there is not a minimal threshold for the transplantation amount of PBSCs in successful transplantation, transfused CD34+ cells less than 2x106/kg may lead to a failed transplantation. CD34+ cells with a large number may have a negative effect including increasing the incidence of acute/chronic graft versus host diseases (GVHD), while may have a positive effect including increasing the survival rate of the patients. Transplantation can be affected by cell subtypes especially T cells and CD14+ mononuclear cells. Transplantation can also be affected by the variation in the quality and quantity of PBSCs.

25% patients, especially those suffered from lymphoma, multiple myeloma and acute leukemia, and 10%-20% normal donors respond poorly to G-CSF, and therefore more components are needed to be removed. Besides, cancer patients receiving extensive treatment and patients suffering from genetic defects such as Fanconi anaemia barely respond to G-CSF, either. Latest studies on PBSC mobilization and autologous transplantation for treating aplastic anemia show that collecting PBSCs by G-CSF is applicable in small portion of patients, and there are wide individual differences among the donors and patients. To extend the application of allogenic transplantation especially in severe patients who are transplanted with paired grafts, patients who are transplanted with tolerogenic organs and patients who have nonfatal genetic defects, more stem cells are required for mobilization to overcome HLA barrier so as to achieve a permanent stem cell implantation, promote activity recovery of white blood cells (WBCs), reduce incidence of GVHD. To date, HSC transplantation has become one of the major treatments for leukemia. However, expensive therapeutic costs deprive hope of most patients suffering from leukemia. There are 40,000-50,000 patients per year newly diagnosed to be suffering from leukemia in our country, and nearly 1 million patients accumulating those in the past years need HSC transplantation therapy. However, 300,000 RMB costs for HSC transplantation are too much for an ordinary family. At present, the common method for clinical stem cell collection includes collecting the HSCs from donors having a kinship with the patients by using G-CSF to mobilize hematopoietic stem/progenitor cells from BM into peripheral blood. However, the procedure for this method is very complicated, and sometimes a surgery is required to meet the quantity demand for transplantation. This is because G-CSF mobilizes hematopoietic stem/progenitor cells indirectly by inducing release of a metalloprotease, which in turn disrupts the interaction of CXCR4/SDF-1, thereby finally mobilizing stem cells. Stem cell donors have to take a drug injection for 4-6 days. Meanwhile, coordination of different departments and professionals in the hospital is also needed.

Although G-CSF has been successfully applied, there are still many problems needed to be solved to achieve manifest clinical breakthrough, including stem cell dose, optimized induction and individual difference for different patients, components to be removed, best mobilizing time and control on the transplanting parts, etc.

BM can contribute to tissue repair as an adult stem cell reservoir. The problems are whether the mobilizing agents can mobilize other stem cells besides HSCs, and whether there are new agents that can specifically mobilize the adult stem cells.

Saturated amine compound is a new type of pharmaceutical intermediate (Rozenbaum W, Antimoniotungstate (HPA-23) treatment of three patients with AIDS and one with prodrome, Lancet, 1985,1:450-451) and has been catalogued in the small molecule compound library. It is a hydrogen bond receptor, and interacts with proteins and carbohydrates via hydrogen bond and special spatial sites. It has been applied against entrance of HIV virus into T cells, inflammation, rheumatoid arthritis, asthma and blood vessel damage, etc.

US2006/281712A1 (Yen Chi-Feng et al., 14 December, 2006) discloses the use of saturated amines for mobilizing hematopoietic stem cells into peripheral blood. The mobilizing agents described in US2006/281712A can be in a form of injection.

### Summary of invention

The present invention is defined in the appended claims and limited thereto. The invention provides a new use of a saturated amine compound, named as TA01, in the manufacture of peripheral blood hematopoietic stem cell mobilizing agents used for HSC transplantation.

The compound TA01 has a structural formula represented by formula I , 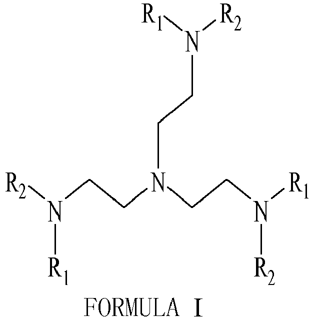 wherein, R₁ = H, CH₃, CH₂CH₃, and R₂ = H, CH₃, CH₂CH₃; preferably, R₁ = R₂ = CH₃ (i.e., Tris[2-(dimethylamino)ethyl]amine)).

Specifically, the invention provides a use of the above compound in mobilizing HSCs into peripheral blood by elevating CD34+ cells and white blood cells in peripheral blood and increasing colony-forming unit (CFU) and colony-forming unit-granulocyte macrophage (CFU-GM) generated by mononuclear cells (MNCs) in peripheral blood.

Mobilizing agents in the invention can be used in a form of injection.

Said injection may adopt various mediums, such as TE buffer (pH 7.5-8.5), physiological saline, phosphate buffered saline (PBS) or sterilized distilled water, etc., preferably, TE buffer (pH7.5-8.5).

One or more pharmaceutically acceptable carriers can be added to the mobilizing agents above when necessary, including conventional diluents, absorption enhancers, surfactants, etc.

Generally, the mobilizing agent above is used in a dosage of 5 mg/kg weight/day, and the course of treatment is 1-2 days. Dosage and course of treatment can be adjusted according to actual conditions.

The invention further comprises a use of the compound in manufacture of agents that can increase percentage of CD34+ cells, numbers of WBCs, CFU and/or CFU-GM generated by MNCs in peripheral blood.

The invention provides a use of a saturated amine compound TA01 in manufacture of peripheral blood hematopoietic stem cell mobilizing agents for HSC transplantation. Experiments show that this compound, being a new kind of mobilizing agent, can both increase percentage of CD34+ cells and number of WBCs in peripheral blood and efficiently mobilize HSCs into peripheral blood. TA01 can mobilize a large quantity of HSCs, and PBSC collection is more convenient in which anesthesia is unnecessary and pain caused by puncture and bone marrow aspiration at multiple sites is also prevented. For patients suffering from partial bone marrow invasion and multiple myeloma, PBSCs can be collected since the bone marrow cannot be collected due to destructed bone, tumor cell infiltration, pelvis radiotherapy, etc. The hematopoiesis is recovered fast post transplantation, complications including infection, hemorrhage occur rarely post transplantation, application of antibiotics and transfusion of blood components are reduced, transplantation related mortality is lowered, length of hospital stay is shortened, and cost is saved. On the other hand, there are less tumor cells in peripheral blood as compared to bone marrow, which in turn lowers the risk of transplantation. The invention can play an important role in research and development of agents for mobilizing HSCs into peripheral blood and has a promising application perspective.

### Brief Description of the Drawings

Figure 1 is a schematic flow chart of the synthesis of TA01.
Figure 2 shows results that TA01 can increase percentage of CD34+ cells in peripheral blood of mice.
Figure 3 shows results that TA01 can increase numbers of WBCs in peripheral blood of mice.
Figure 4 shows results that TA01 can increase both CFU and CFU-GM generated by MNCs in peripheral blood of mice.
Figure 5 shows results that MNCs in peripheral blood mobilized by TA01 can promote the survival rate of the mice receiving a lethal irradiation.

### Detailed Description of the Invention

The invention provides a use of a saturated amine compound TA01 in manufacture of peripheral blood hematopoietic stem cell mobilizing agents for hematopoietic stem cell transplantation. In the hematopoietic stem cell transplantation, mobilizing agents are those supplied to hematopoietic stem cell donors to produce enough hematopoietic stem cells for recipients. The invention provides agents that mainly mobilize hematopoietic stem cells into peripheral blood.

The methods used in following examples, unless otherwise particularly stated, are conventional methods.

### Example 1: Synthesis of TA01

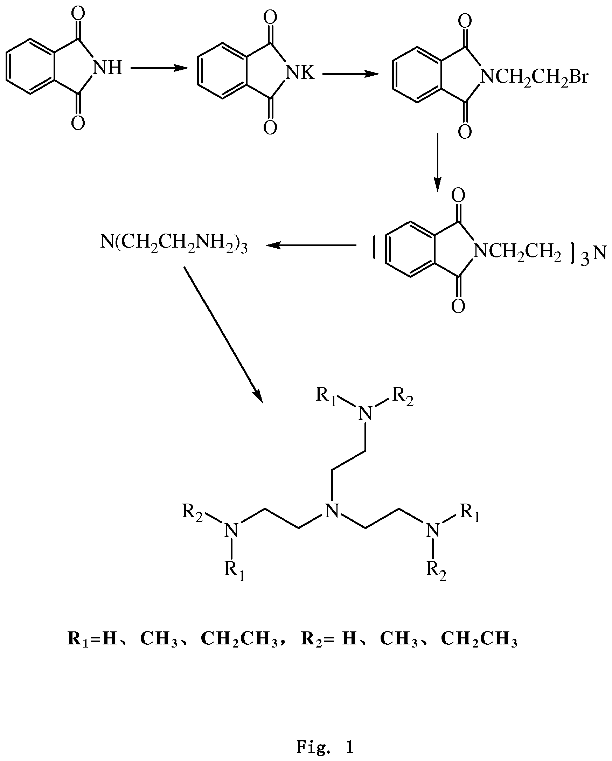

TA01 is synthesized following the procedure shown in Figure 1. Reference: ①Mei GQ, An Improved Method For the Preparation of β,β',β"-Triaminotriethylamine, Journal of Gannan Teachers College, 2002, 3: 55-56; ②Kimura E, et al., Cleavage of amino acid esters and peptides with hydroxoaque (2,2',2"-triaminotriethylamine)cobalt(III)ion, Inorg.Chem, 1970, 9(5): 1187. The method specifically includes the steps of:

### Synthesis of Potassium Phthalimide (I)

To a round bottom flask loaded with 1600 ml anhydrous ethanol, 80.0 g phthalimide was added. The content was heated and refluxed for 0.5h until only a small quantity of raw material was not dissolved. The hot solution was decanting into 30.5 g KOH solution (30 ml H2O+90 ml ethanol), and immediately a white precipitate was generated. The resultant was filtrated after being cooled, and the ethanol was recycled. After being washed with 200 ml acetone, a product was obtained with a weight of 98.0 g and a yield of 98.0%.

### Synthesis of N-β-Bromoethyl-phthalimide (II)

150 g potassium phthalimide and 450 g 1,2-dibromoethane were mixed in a round bottom flask equipped with an agitator and a reflux condensing tube. The round bottom flask was heated for 12 h in an oil bath at a temperature of 180-190°C. Then, the resultant was distilled under reduced pressure to recycle the excessive 1,2-dibromoethane with a weight of 290 g via the condensing tube. 30 ml ethanol was added to the above obtained product, i.e., the mixture of the crude N-β-Bromoethyl-phthalimide and KBr, which were then refluxed for 0.5 h until the black oily substance was completely dissolved. The resulting mixture was filtrated before being cooled and the KBr precipitate was washed with a small quantity of hot ethanol. The filtrates were collected together, and distilled under reduced pressure to remove ethanol.

The above dry remains were added into 500 ml CS2, refluxed for 15-20 mins, and filtrated before being cooled. The filtrate was distilled under reduced pressure to remove CS2, obtaining 131.0 g beige crystal at a yield of 63.6% with a melting point of 78-80°C.

### Synthesis of β,β',β"-triphthalimidetriethylamine (III)

46.0 g product (II) was hot melted and maintained at 140-150°C. Excessive dry NH3 was charged to react with the product(II) for 5-8h. 450 ml ethanol was added to the above mixture, and the new mixture was heated and refluxed for 0.5 h. After being filtrated, the precipitate was washed with hot ethanol, then with a small quantity of water. Glacial acetic acid was used for recrystallisation to obtain 25.2 g white crystals at a yield of 78.0% with a melting point of 187.5°C.

### Synthesis of β,β',β"-triaminotriethylamine hydrochloride (IV)

50 ml concentrated HCl was added dropwise to 20.0 g product (III), and the mixture was reacted for 2 h at a temperature of 150°C. The resultant was concentrated until the volume of the resultant was half of the original volume, and then filtrated to remove the phthalic acid precipitate. The filtrate was treated with hot ethanol having a volume 4 times that of the filtrate and stood for 24 h to precipitate 8.0 g white crystals at a yield of 84.0% with a melting point of 283°C.

### Synthesis of β,β',β"-triaminotriethylamine (V)

6.0 g product (IV) was uniformly mixed with 2.6 g KOH (a molar ratio of 1:2) under oscillation. A small quantity of K2CO3 was added to absorb H2O, and thus amine was separated from the aqueous phase, emitting a strong pungent smell. Then the container was corked. After 24 h, the resultant was filtrated, and two layers of filtrate were collected from a separatory funnel, wherein amine was in the yellowish solution on the upper layer. Said solution was distilled under reduced pressure to obtain a clear transparent liquid. The liquid was dried over metallic sodium and then distilled under reduced pressure. A fraction at 135°C/9 mm Hg was collected to obtain 1.6 g colorless thick liquid at a yield of 47.2%.

### Synthesis of tri(2-(dimethylamino)ethyl)amine (Me6TREN) (VI)

Compound VI was harvested by methylation of compound V.

The synthesized saturated amine compound was verified to be Me6TREN and used as an example in the following experiments. This compound can also be synthesized by methods in other published literatures.

Other compounds represented by formula I can also be synthesized by methods in other published literatures, or by changing some corresponding groups with reference to the above procedures, which are not described herein.

### Example 2: TA01 can increase the percentage of CD34+ cells in peripheral blood

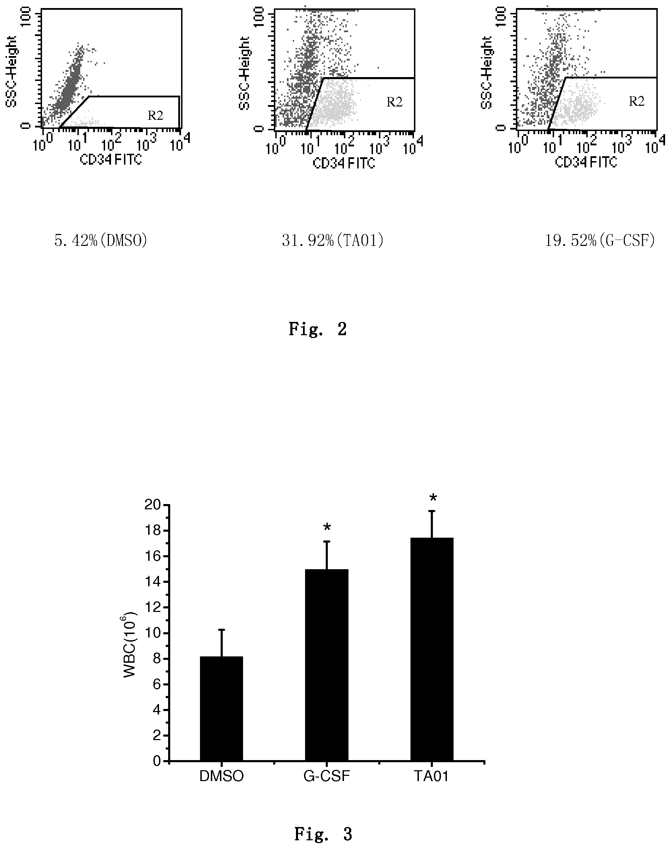

9 C57 mice (Experimental animal center, Academy of Military Medical Sciences), male, were randomly divided into 3 groups: DMSO (Beijing Chemical Inc.) group with 3 mice, G-CSF (Xiamen Baote Biotechnology Inc.) group with 3 mice, TA01 group with 3 mice. Injection volume was calculated on the basis of 5mg/kg body weight, and the medium for the injection was TE buffer with pH 7.5-8.5. At 4 h post hypodermic injection, 100 µL blood was harvested from tail and treated with RBC lysis buffer (Jingmei Biotechnology Inc.), wherein, the volume ratio of the blood to the RBC lysis buffer was 1:10. The two were uniformly mixed, stood for 10-15 min under room temperature, and then centrifuged for 6 min at 1500 rpm/min. After removing the supernatant, 100 ul 1% (v/v) serum (Hyclone Inc.) was added and mixed. Then 0.75 ul purified anti-mouse CD34 (Biolegend Inc) was added (operated on a superclean bench), mixed, and incubated for 1-2 h under room temperature. The mixture was added up to 1.5 ml with PBS containing 1% (v/v) serum, and then centrifuged for 5 min at 1500 rpm/min. After removing the supernatant, 100 ul 1% (v/v) serum was added and mixed. Then 2 ul Goat anti Mice antibody labeled with FITC (Beijing Zhongshanjinqiao Biotechnology Limited Co.) was added, mixed and incubated for 30 min under room temperature. The mixture was added up to 1.5 ml with PBS containing 1 %(v/v) serum, and then centrifuged for 5min at 1500 rpm/min. After removing the supernatant, 500 ul 1 %(v/v) serum was added and mixed. The mixture was assayed by a flow cytometry. It can be seen from the results shown in Figure 2 that TA01 can increase the percentage of CD34+ cells in peripheral blood compared to DMSO and G-CSF.

### Example 3: TA01 can increase the number of WBCs in peripheral blood

9 C57 mice, male, were randomly divided into 3 groups: DMSOgroup with 3 mice, G-CSF group with 3 mice, and TA01 group with 3 mice. Injection volume was calculated on the basis of 5mg/kg body weight, and the medium for injection was TE buffer with pH 7.5-8.5. At 4h post hypodermic injection, 10 µl blood was harvested from tail and treated with 2 ml cell diluting buffer (Jinnan Bolai Biotechnology Limited Co.). The number of WBCs was determined by a Sysmex Microcell Counter. It can be seen from the results (shown in Figure 3, * represents that there was a significant difference as compared with DMSO group) that TA01 can increase the number of WBCs in peripheral blood compared to DMSO and G-CSF.

### Example 4: TA01 can promote the numbers of both CFU and CFU-GM (colony-forming unit-granulocyte macrophage) generated by MNCs in peripheral blood of mice.

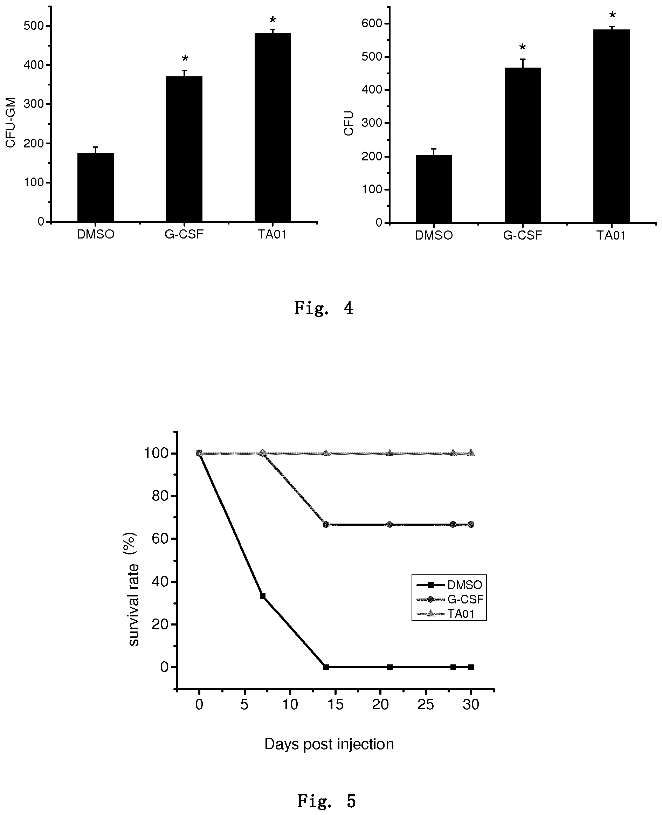

9 C57 mice, male, were randomly divided into 3 groups: DMSO group with 3 mice, G-CSF group with 3 mice, TA01 group with 3 mice. Injection volume was calculated on the basis of 5mg/kg body weight, and the medium for injection was TE buffer with pH 7.5-8.5. At 4h post hypodermic injection, the eyeballs were extracted and the peripheral blood was collected into a 10 ml centrifuge tube which was rinsed with heparin (Tianjin Haoyang Bioproduct Technology Limited Co.) in advance, and then PBS (Hyclone Inc.) was added up to 5 ml and mixed. Mouse percoll (Tianjin Haoyang Bioproduct Technology Limited Co.) was preloaded into vials at 5 ml/vial (operated on a superclean bench), and then the blood mixed with PBS was slightly impregnated into the upper layer of the percoll and centrifuged for 20 min at 2000 rpm/min. Cells in the middle layer were transferred into a new 10 ml centrifuge tube which was filled up to 10ml with PBS, and centrifuged for 5 min at 1500 rpm/min to remove PBS in the supernatant. The tube was filled up to 10 ml with PBS, centrifuged for another 5 min at 1500rpm/min to remove PBS in the supernatant. Then, the tube was filled up to 1 ml with PBS. The number of cells was then counted and the number of the mononuclear cells (MNCs) per ml of the solution was calculated. The resultant was then mixed with 500 µl semisolid medium (components: 0.9% methylcellulose, 15% fetal bovine serum , 100 U/ml penicillin/streptomycin, 1 mM sodium pyruvate, 2 mM glutamine, 2 mM PFHMII, 200 µg/ml transferrin, 1% BSA, 0.45 mM MTG, 30% IMDM, 50 ng/ml SCF, 10 ng/ml TPO, 10 ng/ml IL-3, 10 ng/ml IL-11, 10 ng/ml GM-CSF, 3 U/ml EPO), and cultured for 7 days in an incubator with 5% CO₂ at 37°C. Finally, CFU and CFU-GM were counted. It can be seen from the results shown in Figure 4 that TA01 can promote the numbers of both CFU and CFU-GM generated by MNCs in peripheral blood of mice compared to DMSO and G-CSF (* represents that there was a significant difference as compared with DMSO group).

### Example 5: MNCs in peripheral blood mobilized by TA01 can promote the survival rate of the mice receiving a lethal irradiation

9 C57 mice, male, were randomly divided into 3 groups, and then received a whole body irradiation by ⁶⁰Co γ ray at 2.72 Gy/min with a volume of 7.0 Gy. 9 C57 mice, male, were randomly divided into 3 groups: DMSO group with 3 mice, G-CSF group with 3 mice, and TA01 group with 3 mice. Injection volume was calculated on the basis of 5mg/kg body weight, and the medium for injection was TE buffer with pH 7.5-8.5. At 4h post hypodermic injection, the eyeballs were extracted and peripheral blood was collected into a 10 ml centrifuge tube which was rinsed with heparin in advance, and then PBS was added up to 5 ml and mixed. Mouse percoll (Tianjin Haoyang Bioproduct Technology Limited Co.) was preloaded into vials at 5ml/vial (operated on a superclean bench), and then the blood mixed with PBS was slightly impregnated to the upper layer of the percoll and centrifuged for 20 min at 2000 rpm/min. Cells in the middle layer were transferred into a new 10 ml centrifuge tube which was filled up to 10 ml with PBS, centrifuged for 5 min at 1500rpm/min to remove PBS in the supernatant. The tube was filled up to 10 ml with PBS, and centrifuged for another 5 min at 1500 rpm/min to remove PBS in the supernatant. Then the tube was filled up to 0.5 ml with PBS. The obtained MNCs were then injected into mice suffered from a lethal irradiation by tail vein injection. The number of survival mice in 30 days was counted. It can be seen from the results shown in Figure 5 that the survival rate of the mice receiving a lethal irradiation can be increased by injecting MNCs in peripheral blood mobilized by TA01, compared to DMSO and G-CSF.

The above experiments demonstrated that the compound represented by formula I can efficiently mobilize hematopoietic stem cells into peripheral blood with tri(2-(dimethylamino)ethyl)amine as an example. The invention also verified that other compounds represented by formula I produced the similar effects, the verification processes of which are not described herein.

### Industrial Applicability

The invention provides a new use of a saturated amine compound TA01 in manufacture of peripheral blood hematopoietic stem cell mobilizing agents for hematopoietic stem cell transplantation. Experiments show that this compound can efficiently mobilize hematopoietic stem cells into peripheral blood, and is a new type of hematopoietic stem cell mobilizing agents. The invention can play an important role in research and development of mobilizing agents for mobilizing hematopoietic stem cells into peripheral blood and has a promising application perspective.

## Claims

1. A use of a saturated amine compound having a chemical formula represented by formula I in the manufacture of peripheral blood hematopoietic stem cell mobilizing agents for hematopoietic stem cell transplantation: 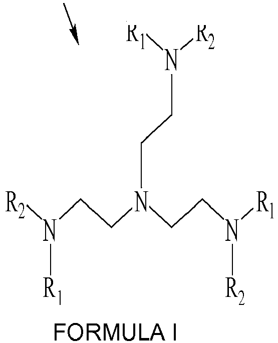 Wherein, R1 = H, CH₃ or CH₂CH₃, and R2 = H, CH₃, or CH₂CH₃.

2. The use according to claim 1, wherein R1 = R2 = CH₃, and the compound is tri(2-(dimethylamino)ethyl)amine.

3. The use according to claim 1, wherein the mobilizing agents are in a form suitable for injection.

4. The use according to claim 3, wherein a medium for the injection is TE (Tris-EDTA) buffer with a pH of 7.5 - 8.5, physiological saline, phosphate buffered saline (PBS) or sterilized distilled water.

5. The use according to claim 1 or 2 in a manufacture of agents for increasing the percentage of CD34⁺ cells in peripheral blood.

6. The use according to claim 1 or 2 in a manufacture of agents for increasing the number of white blood cells in peripheral blood.

7. The use according to claim 1 or 2 in a manufacture of agents for increasing the numbers of colony-forming units and/or colony-forming unit-granulocyte macrophages generated by mononuclear cells in peripheral blood.

8. The use according to claim 1 or 2 in a manufacture of hematopoietic stem cell mobilizing agents for increasing the percentage of CD34+ cells, and numbers of white blood cells, colony-forming units and colony-forming unit-granulocyte macrophages generated by mononuclear cells in peripheral blood.

9. A peripheral blood hematopoietic stem cell mobilizing agent comprising the saturated amine compound of claim 1 for use in hematopoietic stem cell transplantation.

## Patentansprüche

1. Verwendung einer gesättigten Aminoverbindung mit einer chemischen Formel nach FORMEL 1 bei der Herstellung von peripheren blutbildenden hämatopoetischen Stammzellen, die Mittel zur hämatopoetischen Stammzelltransplantation mobilisiert: 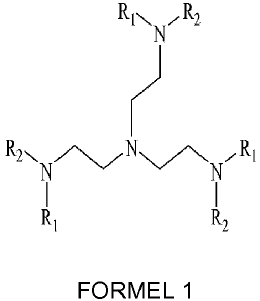 wobei R1 = H, CH₃ oder CH₂CH₃ und R₂ = H, CH₃ oder CH₂CH₃ ist.

2. Die Verwendung nach Anspruch 1, wobei R1 = R₂ = CH₃ und die Verbindung Tri(2-(dimethylamino)ethyl)amin ist.

3. Die Verwendung nach Anspruch 1, wobei die Mobilisierungsmittel für eine Injektion geeignet sind.

4. Die Verwendung nach Anspruch 3, wobei ein Mittel für die Injektion Tris-EDTA Puffer mit einem pH von 7.5 - 8.5, physiologische Kochsalzlösung, phosphatgepufferte Salzlösung (PBS) oder sterilisiertes destilliertes Wasser ist.

5. Die Verwendung nach Anspruch 1 oder 2 bei der Herstellung von Mitteln zur Erhöhung des Prozentsatzes von CD34+ Zellen in peripherem Blut.

6. Die Verwendung nach Anspruch 1 oder 2 bei der Herstellung von Mitteln zur Erhöhung der Anzahl weißer Blutkörperchen in peripherem Blut.

7. Die Verwendung nach Anspruch 1 oder 2 bei der Herstellung von Mitteln zur Erhöhung der Anzahl koloniebildender Einheiten und/oder koloniebildender Einheits-Granulozyten Makrophagen erzeugt durch mononukleäre Zellen im peripheren Blut.

8. Die Verwendung nach Anspruch 1 oder 2 bei der Herstellung von Mobilisierungsmitteln für hämatopoetische Stammzellen zur Erhöhung des Prozentsatzes an CD34+ Zellen und der Anzahl von weißen Blutkörperchen, koloniebildenden Einheiten und koloniebildender Einheits-Granulozyten Makrophagen, die durch mononukleäre Zellen im peripheren Blut erzeugt sind.

9. Mobilisierungsmittel für hämatopoetische Stammzellen, umfassend die gesättigte Aminoverbindung nach Anspruch 1 zur Verwendung bei hämatopoetischer Stammzellentransplantation.

## Revendications

1. - Utilisation d'un composé amine saturée ayant une formule chimique représentée par la formule I dans la fabrication d'agents de mobilisation de cellules souches hématopoïétiques du sang périphérique pour une greffe de cellules souches hématopoïétiques : 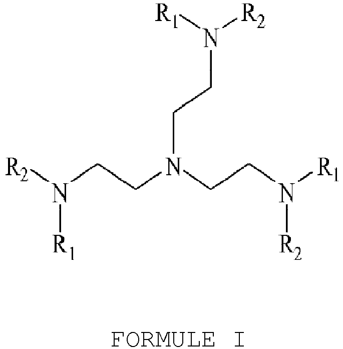 dans laquelle R1 = H, CH₃ ou CH₂CH₃, et R2 = H, CH₃ ou CH₂CH₃.

2. - Utilisation selon la revendication 1, dans laquelle R1 = R2 = CH₃, et le composé est la tri(2-(diméthylamino)éthyl)amine.

3. - Utilisation selon la revendication 1, dans laquelle les agents de mobilisation sont sous une forme appropriée pour une injection.

4. - Utilisation selon la revendication 3, dans laquelle un milieu pour l'injection est un tampon TE (Tris-EDTA) ayant un pH de 7,5 - 8,5, une solution saline physiologique, une solution saline tamponnée au phosphate (PBS) ou de l'eau distillée stérilisée.

5. - Utilisation selon l'une des revendications 1 ou 2 dans une fabrication d'agents pour augmenter le pourcentage de cellules CD34+ dans le sang périphérique.

6. - Utilisation selon l'une des revendications 1 ou 2 dans une fabrication d'agents pour augmenter le nombre de globules blancs dans le sang périphérique.

7. - Utilisation selon l'une des revendications 1 ou 2 dans une fabrication d'agents pour augmenter le nombre d'unités de formation de colonies et/ou d'unités de formation de colonies de granulocytes macrophages générées par des cellules mononucléaires dans le sang périphérique.

8. - Utilisation selon l'une des revendications 1 ou 2 dans une fabrication d'agents de mobilisation de cellules souches hématopoïétiques pour augmenter le pourcentage de cellules CD34+, et le nombre de globules blancs, d'unités de formation de colonies et d'unités de formation de colonies de granulocytes macrophages générées par des cellules mononucléaires dans le sang périphérique.

9. - Agent de mobilisation de cellules souches hématopoïétiques du sang périphérique comprenant le composé amine saturée selon la revendication 1, pour une utilisation dans une greffe de cellules souches hématopoïétiques.
